# EUROPEAN PATENT SPECIFICATION

(11) **EP 0 658 339 B1**
(45) Date of publication and mention of the grant of the patent: **07.06.2000**
(21) Application number: 94308892.2
(22) Date of filing: 30.11.1994
(51) Int. Cl.: A61K 7/13

(54) **Process and kit for dyeing hair**
Verfahren und Kit zum Färben der Haaren
Procédé et kit pour la teinture des cheveux

(30) Priority: 30.11.1993 US 159988
(43) Date of publication of application: 21.06.1995
(73) Proprietor: Bristol-Myers Squibb Company, New York, N.Y. 10154 (US)
(72) Inventor: Prota, Giuseppe, I-80127 Naples (IT); Wenke, Gottfried, Woodbridge, Connecticut 06525 (US)
(74) Representative: Cropp, John Anthony David

(56) References cited:
- WO-A-93/05759
- FR-A- 2 252 841

## Description

The present invention relates to a hair dyeing process employing a melanin precursor formed by reaction of a dopa species, an oxidant and a direct dye. In addition, the present invention concerns a method of dyeing hair wherein the melanin is generated by the user from separately packaged reactants sold in the form of a kit.

### BACKGROUND OF THE INVENTION

As reported, for example, in Prota, Progress in the Chemistry of Melanins and Related Metabolites, Med. Res. Reviews, 8:525-56 (1988), melanins are naturally occurring pigments present in hair and skin. In humans, biosynthesis takes place in tyrosinase containing melanocytes. The tyrosinase enzyme catalyzes the hydroxylation of tyrosine to dopa and its subsequent oxidation to dopachrome. Once formed, dopachrome undergoes a series of complex reactions in the formation of eumelanins and phaeomelanins.

Melanins provide black and deep brown pigments, and are formed by oxidative polymerization of 5,6-dihydroxyindole derived biogenetically during the melanogenesis. On the other hand, phaeomelanins provide yellow to reddish brown pigmentation to hair and are formed by oxidative polymerization of cystein-S-yl-dopas via 1,4-benzothiazine intermediates.

Synthetic 5,6-dihydroxyindole (DHI) has been disclosed in the prior art for use in hair and skin dyeing. For example, U.S. 2,934,396 to Charle discloses a process for dyeing hair by contacting hair with an aqueous solution of DHI having a pH of at most 7 for 5 to 60 minutes, followed by an application of an aqueous solution capable of inducing oxidation and/or polymerization of DHI.

Dopa and dopamine are disclosed as hair dyeing precursors in the process of Herlihy, U.S. Patent 4,746,322, wherein the aqueous hair dyeing composition comprises said precursor, an organic compound to assist dye dispersion and an iodate or periodate. The dopa or dopamine dye precursor is present in the aqueous hair dye composition in an amount of from about 1 to about 100 mg/ml, preferably from about 5 to about 25 mg/ml. Dopamine is preferred, according to Herlihy. The iodate or periodate is present in the composition at a concentration of 1 to about 50 mg/ml, while the dispersing agent is present in an amount of from about 0.1 to 30% (wt./vol.). Optionally, a color modifier can be incorporated into the aqueous composition of Herlihy, at a level of from about 0.1 to about 10 mg/ml. pH may be maintained between about 3 to about 7 by incorporation of an effective amount of a buffer. According to Herlihy, the above-described aqueous compositions disperse the dye on the hair shaft "with little or no penetration into the hair shaft." Column 2, lines 56-58.

The prior art fails to provide a commercially feasible process for effectively, permanently dyeing hair using dopa as a starting reagent. It is believed this failing is attributable to an inability of the prior art processes in making a melanin precursor available on the hair at concentrations suitable for its diffusion into the hair, for subsequent conversion to nondiffusable melanin, as further explained in detail below.

Indeed, the inability to provide an inexpensive yet effective process for dyeing hair with a melanin precursor has prevented use of melanogenesis in the commercial dyeing of hair.

Interest in melanogenesis to dye hair is quite high, however. This is because synthetic melanin pigments provide an exceptionally natural-looking deep brown or black color. Moreover, they are not irritating to the skin. Nor are they mutagenic.

WO93/05759 describes a process for dyeing hair by contacting the hair with a preformed aqueous medium comprising a dopa species, an oxidant and a buffer to maintain the pH in the range of from 6 to 10. The composition may additionally contain a primary intermediate or coupler.

It has now been found, quite surprisingly, that an aqueous hair dyeing process wherein an effective melanin-forming hair dyeing amount of 5,6-dihydroxyindole is generated during the reaction of dopa with an oxidant can be practiced inexpensively and under commercially feasible conditions, to achieve a permanent hair color. The process involves including a reactive direct dye in the reaction mixture.

By means of the present invention, an aqueous hair dyeing process, wherein an effective melanin-forming hair dyeing amount of a nitrogenous phenolic, especially indolic, melanin precursor is generated during the reaction of select substituted dopa compounds with an oxidant, can be practiced inexpensively and under commercially feasible conditions, to achieve a permanent hair color. Advantageously, the utilization of the substituted dopa compounds of the present invention is conducive to the attainment of a range of hair color shades, in contrast to the use of dopa alone as the starting reagent, which is capable merely of providing gray or black pigmentation to hair.

It has additionally been found that even further and more desirable color modifications to hair dyed in accordance with the process of the present invention may be obtained by including in the reaction mixture, along with the dopa and/or substituted dopa compound, one or more hair dye compounds selected from the group consisting of direct dyes, primary intermediates and couplers.

The hair dyeing process of the present invention contemplates the preparation of an aqueous hair dyeing composition by reacting dopa or a selected substituted dopa compound as hereinafter defined, with a ferricyanide oxidant and a reactive direct dye to form a melanin-forming hair dye precursor, and applying the aqueous composition to the hair. The melanin precursor contained in said aqueous composition is capable of diffusing into the hair shaft in an amount effective to dye hair permanently upon its coincident conversion to melanin while in the hair.

The aqueous hair dyeing composition is produced by initiating reaction between the dopa species or a salt thereof, the direct dye and with an inorganic oxidant that is a soluble ammonium, alkali or alkaline earth metal salt, especially sodium and potassium salts, of ferricyanide in an aqueous reaction medium buffered by sufficient buffering agent to maintain the reaction medium pH from about 6 to about 10 throughout the series of reactions that take place leading to the melanin precursor.

In order to achieve the permanent dyeing of hair in accordance with the process of the present invention, it is critical to generate melanin from the melanin-forming hair dye precursor in the aqueous hair dye composition in such amount as to effect a color change to the hair. The total color change may be gradually obtained by several applications of the composition over time, or may be effected by a one-time application of the composition, depending on the concentration of the dopa species, the duration of application, and the desires of the user. It is further critical that the hair dye composition be applied to the hair prior to the substantial formation of melanin so that the melanin precursor formed during the reaction may diffuse into the hair prior to the generation of melanin, the melanin then being formed within the hair. It is additionally important that the process for dyeing hair as described herein be capable of completion within less than about one hour.

In the case in which the dopa species is dopa or a salt thereof, the reaction with the oxidant leads to the formation of 5,6-dihydroxyindole, which melanin precursor, upon its conversion to melanin, provides hair with a permanent black color. In the case of the selected substituted dopa compounds, melanin precursors are obtained which, upon conversion to melanin, produce a range of shades depending upon the selection of the substituted dopa compound.

A further aspect of the invention is the optional incorporation of a hair dye component selected from the group consisting of direct dyes, primary intermediates, couplers and mixtures thereof in the reaction mixture. Following the initial dopa species-oxidant reaction, it is believed that the direct dyes and optional primary intermediate(s) and/or coupler(s) present in the reaction mixture react at least in part with the intermediate compounds formed prior to the melanin precursor, thereby providing chromatic characteristics to the melanins ultimately obtained.

In another aspect of the present invention, it has been found that the formation of indolic melanin precursors such as 5,6-dihydroxyindole is hastened by proper selection and amount of the buffer, apart from its requirement for maintaining pH of the reaction medium. Preferably, the buffer is a phosphate, carbonate or bicarbonate, and typically is included in substantial excess over the amount needed to maintain the requisite pH.

In yet another aspect of the present invention, the process for dyeing hair contemplates treatment of the hair with agent(s) that promote melanin formation, e.g., a solution of a metal ion salt, which treatment accelerates the formation of melanin from the ultimate indolic precursor. Treatment with the promoting agent may be a pre- or a post-treatment, or in some instances may be conducted simultaneously with the application of the hair dye composition of the present invention.

The process of the present invention may conveniently be practiced by providing premeasured amounts of the reactants in separate containers packaged in kit form. The user simply admixes the reactants on or with subsequent application to the hair and allows the composition while it is reacting to remain on the hair for the prescribed period of time. It is seen that no special expertise is required to carry out the process, and accordingly the product and process are equally suitable for in-home use by the nonprofessional as well as salon use by the professional. Advantageously, the product in kit form is shelf-stable and is therefore suitable for retail sale and without precautions generally required for melanin-forming precursors, such as 5,6-dihydroxyindole, eg., storage under anaerobic conditions.

According to the present invention, there is provided a method of permanently dyeing hair with a melanin precursor characterised in that it comprises the steps of:
(a) forming a melanin precursor by reacting a composition containing a dopa species and a reactive direct dye with a hair dye component which is an oxidant selected from soluble ammonium, alkali metal and alkaline earth metal ferricyanide salts in an aqueous reaction medium containing a buffering agent, the concentrations of the dopa species, reactive direct dye and oxidant reactant being in amounts effective to produce a hair coloring concentration of the melanin precursor in the aqueous reaction medium, said buffering agent being present in the aqueous reaction medium in an amount sufficient to maintain its pH in the range 6 to 10,
(b) contacting the hair with the aqueous reaction medium and allowing the melanin precursor to diffuse into the hair in an amount sufficient to generate a hair coloring amount of melanin, and
(c) permanently coloring the hair by allowing the melanin precursor present in the hair to form melanin.

The hair dyeing process of the present invention comprises the preparation of an aqueous hair dyeing composition by reacting dopa or selected substituted dopa or dopa analogs (hereinafter referred to collectively as the "dopa species"), and an inorganic oxidant, in the presence of a reactive direct dye and, optionally, in the presence of another oxidative hair dye component selected from the group consisting of primary intermediates, couplers, and mixtures thereof and contacting the hair with said hair dyeing composition for a period of time of about less than one hour, said reaction proceeding in such manner and under such conditions as to provide on the hair an amount of a melanin-forming hair dye precursor during the period of contact effective to generate a hair dyeing amount of melanin. The precursor diffuses into the hair during the period of contact and forms melanin in situ in the hair to provide a permanent color. Preferably, the contact time of the hair dyeing composition on the hair is from about 5 to about 45 minutes, most preferably from about 5 to about 30 minutes.

By "permanent" is meant a color not removable by shampooing with a conventional surfactant-containing shampoo, the permanency being attributable to the inability of melanin to diffuse from the hair shaft in view of its molecular size.

By "melanin" is meant a synthetically derived pigment formed by polymerization of a melanin precursor, i.e., the formation of molecules too large to be removed from the hair.

By "melanin-forming precursor" is meant the reaction product(s) of the dopa species with a ferricyanide oxidant and a reactive direct dye, optionally together with a primary intermidiate or coupler hair dye component, which reaction product(s) undergoes polymerization to form melanin. Such melanin precursors generically are nitrogenous phenolic compounds and are indolic compounds, except to the extent that cyclization to form the indole ring might be prevented in view of reactions occurring with direct dyes and, the optional couplers and/or primary intermediates, as hereinafter disclosed.

Applicants herein believe that the terms "melanin" and "melanin precursor" as used herein with respect to the reaction products of the selected dopa species of this invention are terms which are will understood by one or ordinary skill in the field, even though the chemical identity of the melanin precursors, particularly those precursors formed by reaction with direct dyes or, if present, with primary intermediates and/or couplers, and especially the melanins formed in accordance with the process of the present invention, may not be precisely known or understood.

In another aspect of the present invention, (a) the dopa species and reactive direct dye and (b) the oxidant reactant are separately provided in kit form, for admixture by the user to initiate the reaction. It is possible to combine the reactants directly on the hair of the user, but preferable to mix them in a mixing vessel, for subsequent application to hair following commencement of the reaction.

It has been found in accordance with the invention that the color obtained by oxidation of the dopa species can be significantly modified by the inclusion of the direct dye and optional primary intermediates and/or couplers in the reaction medium. In this regard, the terms "melanin precursor" and "melanin" are intended to include reaction products of direct dyes, primary intermediates and couplers with the dopa species and with reaction products of the dopa species produced by oxidation with the oxidant. While such melanin precursors are nitrogenous phenolic compounds, it is not known whether they have an indole ring in their chemical structure.

The hair dyeing process involves a series of reactions leading to the formation of one or more melanin precursors capable of diffusing into the hair shaft. Within the hair shaft, the precursor is oxidized by air to melanin, which is incapable of diffusion out of the hair shaft. Accordingly, the melanin precursor-containing hair dye composition must be applied to the hair prior to the substantial formation of melanin. Inasmuch as the precursor, upon formation, will begin its conversion to melanin by reaction with air, it is critical to apply the reaction medium to hair prior to onset of substantial melanin formation, that is, at or shortly after admixture of the reactants.

The term "applying" as used herein means the contact between the hair dye composition and the hair as described above. Placing the hair dye composition on the hair following substantial melanin formation is not operable since the insoluble melanin will not diffuse into the hair, and will be largely stripped away during subsequent shampooing. For convenience, a contact time of "less than about one hour" as used throughout this application is measured from the onset of mixing of the reactants.

It should also be understood that a suitable aqueous hair dyeing composition can be obtained without adding additional constituents to the aqueous reaction medium. That is, the aqueous reaction medium and the aqueous hair dyeing composition may be regarded as equivalents, for example, in the case where the reactions involved occur, in whole or in part, upon the hair to be dyed. As described below, however, it is preferred to include additional optional constituents, e.g., thickeners, etc., to provide a more elegant product.

In the process of the present invention, the dopa species is oxidized by the oxidant through a series of reactions leading to the formation of one or more melanin precursors. While not wishing to be bound by any particular reaction scheme, applicants herein believe that the following reactions occur leading to the formation of the melanin precursors: (1) oxidation of the dopa species by the ferricyanide oxidant followed by cyclization, further oxidation and rearrangement with carbon dioxide release, leading to the formation of an indole, e.g., the conversion of dopa to 5,6-dihydroxyindole, (2) oxidation of the dopa species followed by cyclization, further oxidation and rearrangement without carbon dioxide release, and (3) reactions wherein the initial dopa species oxidation product(s) is modified by further reaction with a direct dye or with with a coupler or primary intermediate when these are present, leading to nonindolic nitrogeneous phenolic compounds.

In the case of dopa, for example, dopa is oxidized to dopaquinone, which spontaneously forms cyclodopa. Additional oxidant further reacts with the cyclodopa to form dopachrome which undergoes spontaneous, although not immediate, transformation to 5,6-dihydroxyindole through rearrangement of the dopachrome species and the release of carbon dioxide. Analogous reactions also take place with regard to alpha alkyl dopas. Dopa alkyl esters also react similarly, but without release of carbon dioxide. The reactions for the preparation of melanin from dopa in accordance with the present invention are presented below.

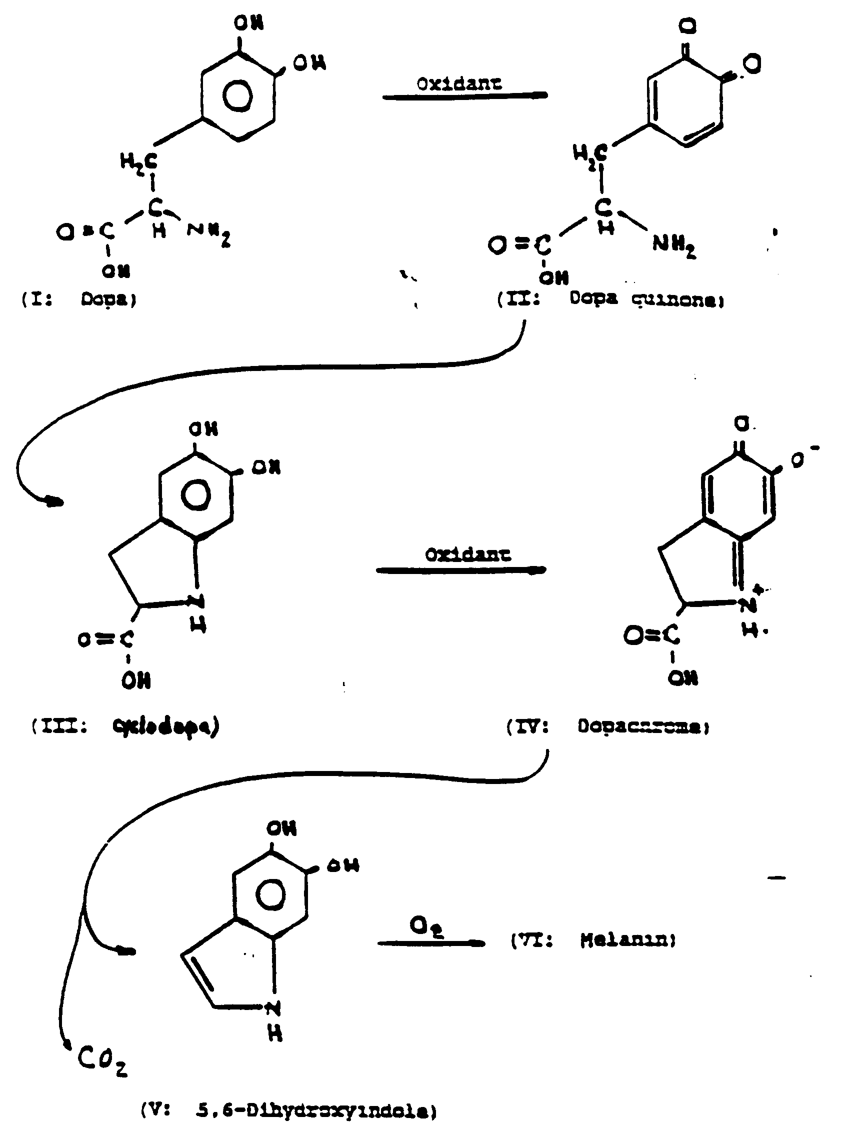

It is seen that the sequence of reactions contemplated in the process of the present invention is conducive to many possible competing reactions. Because second order reactions are likely to be involved, the problem of unwanted competition reactions becomes especially acute when the concentrations of starting reactants in solution are high, as in the process of the present invention.

A second difficulty believed to exist is that the rearrangement of cyclized indolic compounds, when it occurs, for example, in the conversion of dopachrome to 5,6-dihydroxyindole, is the rate-determining step in the reaction leading to the melanin precursor.

Yet another problem that has hitherto mitigated against the commercial use of the dopa species as a starting reagent in the dyeing of hair is that the melanin precursor, which oxidizes relatively slowly in air to form melanin, is essentially immediately oxidized by unreacted oxidant to form by-products unsuitable for permanently dyeing hair.

In overcoming each of these difficulties, the present invention achieves a melanin precursor concentration in the aqueous hair dyeing composition that leads to a melanin level effective for permanently dyeing hair, and provides a process that can be practised by the user in under about 60, preferably under 45, most preferably under 30 minutes.

Thus, the present invention contemplates conversion of the dopa species to the melanin precursor at yields and in amounts effective to color hair permanently. To this end, applicants have found that the ferricyanide oxidant, when present in the reaction media in monitored amount, is conducive to the attainment of melanin precursor concentrations in the dyeing composition effective to dye hair.

Accordingly, in the process of the present invention, the amount of oxidant present in the reaction medium relative to the dopa species should be such that the oxidant is largely reacted prior to the appreciable formation of the melanin precursor.

With regard to the second difficulty, it is believed that the above-mentioned rearrangement step may be accelerated by use of particular buffer constituents in a rate-potentiating concentration, thereby permitting completion of the process within about one hour.

### The Dopa Species

As previously indicated, the preparation of the aqueous hair dye composition is by the consumer, who admixes the reactants at the time of use. The dopa species or a suitable salt thereof is present in the initial reaction medium at a level suitable to obtain a hair dyeing amount of melanin, which melanin amount, in turn, is dependent on the melanin intermediate levels achieved during the period of contact of the hair dyeing composition with the hair.

The required initial dopa species concentration in the reaction medium may be higher than its solubility limit in water. Accordingly, an acid or alkaline aqueous premix can be prepared prior to preparation of the aqueous reaction medium. Alternatively, the more soluble acid or basic salts can be used in the preparation of the aqueous medium. Use of the salts or the use of an acid or alkaline premix allows the otherwise relatively insoluble dopa reactant to go into solution and be available for rapid reaction.

Illustrative of the suitable soluble acid salts of the dopa species are the hydrochloride and sulfate. The hydrochloride salts are preferred. Among the suitable basic salts that can be used are the soluble alkali metal salts and the alkaline earth metal salts. The sodium and potassium salts are preferred. Any inorganic or organic acid or base can be used to adjust the pH of the dopa species premix solution, provided that the agent used does not interfere in the reactions. Suitable bases are ammonium and sodium hydroxide and mono-, di- and trialkanolamines, especially ethanolamines. Such acids are hydrochloric, phosphoric, tartaric, citric and lactic acids and their salts. Sodium hydroxide and hydrochloric acid are preferred.

The dopa species (or dopa species salt) concentration in the initial reaction medium is from about 2 mg/ml up to about the solubility limit of the dopa species in the reaction medium. Preferably, its concentration is from about 5 to about 25 mg/ml in the initial reaction medium, most preferably from about 5 to about 15 mg/ml.

The dopa species is selected from dopa and substituted or analog dopa compounds. Substituted or analog dopa species (referred to herein as the "substituted dopa" species) suitable in the process of the present invention are alpha alkyl dopa having 1 to 4, preferably 1 to 2, carbon atoms in the alkyl groups, epinephrine (adrenaline) and dopa alkyl esters having 1 to 6, preferably 1 to 2, carbon atoms in the alkyl group.

Alpha alkyl dopa is oxidized by the ferricyanide oxidant in analogous manner to dopa, to form 5,6-dihydroxy-2-alkylindole, which forms melanin by aerobic oxidation.

Epinephrine, which has the structure: 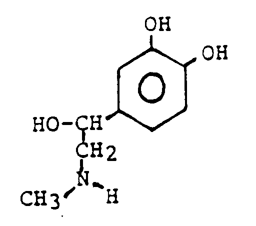 reacts with the ferricyanide oxidant to form adrenochrome. It is believed that adrenochrome rearranges to form adrenolutin and various indolic and/or isatinic derivatives.

In the case of the dopa alkyl esters, oxidation proceeds to form the corresponding esters of 5,6-dihydroxyindole-2-carboxylic acid, which reaction proceeds without decarboxylation, i.e., there is no release of carbon dioxide. This ester of 5,6-dihydroxyindole-2-carboxylic acid then polymerizes to melanin by aerobic oxidation.

### The Oxidant Component

Suitable as the oxidant for use in the present invention is a soluble ammonium, alkali metal and alkaline earth metal salt, especially ammonium, sodium and potassium salt of ferricyanide. Advantageously, the reduced form of ferricyanide -- ferrocyanide -- present in the aqueous solution following the reaction will not further react with the melanin precursor in the aqueous system, thereby maximizing the formation of the melanin precursor and hence increasing the overall efficiency of the process.

The oxidant is quite reactive towards the dopa species present in the reaction medium during the process. Thus, the initial reaction between the dopa species and the oxidant goes essentially to completion within less than five minutes, most likely in less than one minute, and might even be regarded as instantaneous in some instances. For this reason, intermediates in the postulated reaction schemes leading to the formation of the melanin precursor are short-lived in the reaction media and not available for inter-reaction. Accordingly, in the process of the present invention, unwanted side reactions are prevented or greatly limited.

The oxidant reactant is present in the initial reaction medium at a substantially stoichiometric equivalent concentration, as further described below.

During the conversion of the dopa species to the melanin precursor, each dopa species molecule loses four electrons. Accordingly, if an oxidant is employed that gains one electron, such as ferricyanide, four molar equivalents of oxidant are required to convert dopa to dihydroxyindole. Thus, two molar equivalents of ferricyanide are needed to convert dopa to dopaquinone and another two molar equivalents of ferricyanide are required to convert cyclodopa (spontaneously obtained from dopaquinone) to dopachrome.

In the case of dopa, one "stoichiometric equivalent" as used herein is equal to the number of molar equivalents of an oxidant necessary to convert one mole of dopa to one mole of dopachrome (which spontaneously forms dihydroxyindoles). For the substituted dopa species, analogous reactions are believed to occur. Thus, the alkyl dopa species form alkyl dihydroxyindoles, the alkyl dopa esters form dihydroxyindole carboxylic acid esters, and epinephrine is believed to form adrenolutin, each conversion resulting in the loss of four electrons.

A greater than about a stoichiometric equivalent amount of oxidant relative to the dopa species employed is not recommended, as the excess oxidant will react with the melanin precursor. The dopa species (dopa or substituted dopa) in an excess stoichiometric equivalent amount relative to oxidant is preferred to ensure that unreacted oxidant does not remain following the reaction. An excess of the dopa species does not appear to affect the process performance, although unreacted substituted dopa would tend to reduce the overall efficiency of the process. Generally, the stoichiometric equivalent ratio on a molar basis of the dopa species to ferricyanide initially present in the reaction medium will be from about 1.25:1 to 0.95:1, preferably from about 1.1:1 to 1:1, most preferably from about 1.05:1 to 1.01:1.

When the oxidative hair dye components are optionally incorporated in the reaction mixture, it is believed that the direct dye, (and optional primary intermediate and/or coupler hair dye compounds when present) react with one or more of the intermediate reaction products prior to rearrangement of the cyclized intermediate. Further, it is believed that a portion of the dopa species initially present in the reaction medium is reacted to completion to form 5,6-dihydroxyindole or the equivalent analog melanin precursor. Theoretically, then, when the additional oxidative hair dye components are incorporated, the initial reaction medium should contain between two to four molar equivalents, i.e., between 0.5 to 1 stoichiometric equivalents of oxidant relative to the dopa species based on complete conversion of dopa to the melanin precursor. Accordingly, the stoichiometric equivalent ratio on a molar basis of the dopa species to oxidant initially present in the reaction medium is generally from 1:1 to 2:1, preferably from about 1.2:1 to 1.8:1, most preferably from about 1.3:1 to about 1.7:1. The stoichiometric equivalent amount of oxidant relative to dopa should not be so great as to cause an excess of the oxidant to be present after formation of the melanin precursor, because oxidation of the melanin precursor by the oxidant is not desirable.

It might be possible to add oxidant slowly or in stages during the reaction. However, this would be difficult and inconvenient for the consumer, and may inadvertently result in oxidant being present when the melanin precursor is formed.

### The Buffering Agent Component

Inasmuch as the pH of the reaction medium will fall during the reactions, it is necessary to provide a sufficient amount of a buffering agent in the reaction medium to maintain the requisite pH. In the process of the present invention, it is critical to maintain the pH of the aqueous reaction medium in the range 6 to 10 during the melanin precursor-forming. Preferably, the pH is between about 6 to about 8.5, and especially alkaline to about 8.5.

In addition to controlling reaction medium pH within the aforesaid limits, the buffers employed in the process of the present invention are believed to assist in the formation of the melanin precursors. Thus, it has been observed that as the concentration of the buffers in the reaction medium increases, the rate of the rearrangement of dopachrome and its analog also increases. Thus, the buffers potentiate the rearrangement reaction, thereby decreasing the time for the generation of the melanin precursor, which permits the hair dyeing process to be completed within about one hour from the onset of the dopa species oxidant reaction. Typically, the buffer is present in an amount in excess of that needed to buffer the reaction mixture. Preferably, then, it is desirable to provide 2 to 25 times, especially 5 to 20 times, as much of these particular buffers as would be needed merely to maintain the reaction mixture pH within the prescribed limits.

Buffers found to be suitable for use in this invention are ammonium and alkali metal phosphates, bicarbonates, carbonates and, to a lesser extent, borates. Also suitable are aminic buffers such as N-[2-hydroxyethyl]piperazine-N'-[2-ethanesulfonic acid] (HEPES), N-[2-acetamido]-2-aminoethane sulfonic acid (ACES), tris[hydroxymethyl]aminomethane (TRIZMA) and N-trist[hydroxymethyl]-methyl-3-aminopropane sulfonic acid (TAPS). The ammonium and alkali metal carbonates and bicarbonates are suitable, even though not typically employed in the stated pH range. The preferred buffers used in the practice of the present invention are sodium and potassium carbonate, bicarbonate or phosphate when the oxidant is ferricyanide and phosphate buffer with the permanganate oxidant. Other buffers suitable for maintaining reaction medium pH and to potentiate the rearrangement reaction may exist which may be determined by simple experimentation, as herein disclosed in the examples.

### The Process Parameters

It should be understood that the ability to obtain the necessary melanin precursor concentration depends on both its yield and the amount of the dopa species available for conversion. Thus, a lower melanin precursor yield would be acceptable when a high initial dopa species concentration is provided in the reaction medium. Conversely, a relatively high melanin precursor yield would be needed if a low dopa species concentration is used.

In the present invention for permanently dyeing hair, the melanin precursor is converted to melanin in situ while the hair dyeing composition is in contact with the hair. Thus, the process should be viewed as a dynamic one in which the various reactions leading to melanin proceed simultaneously. Accordingly, the concentration and molar yield of the melanin precursor based on the dopa species formed in the hair dye composition is not directly measurable unless the subsequent melanin-forming reaction is prevented. Even then, the measurement of the yield is complex in view of the number of competing reactions and the number of chemical species present. The measurements are especially complex and difficult for the substituted dopa species, in particular, epinephrine, and because the reaction mixture further includes a direct dye. Similarly, amount and yield of melanin is not easily quantitatively measurable because it is formed in the hair. On the other hand, the effectiveness of the process may be determined by measuring the change in hair color when a hair swatch is treated in accordance with the process. Further, such evaluation is an indication of the amount of melanin that has formed in the hair shaft, and hence the amount of precursor that has diffused into the hair shaft during the treatment. The test procedure is discussed further below. As a guide to the successful practice of the invention, applicants have found that a perceptible color change to hair occurs within one hour of application to the hair. A suitable melanin precursor molar yield is typically obtained when the initial dopa species concentration is from about 2 mg/ml up to its solubility limit in the reaction medium.

In the case where dopa alone is contained in the reaction medium (i.e., direct dyes and optional couplers and/or primary intermediates are not present), applicants have found that a perceptible color change to hair occurs within one hour of application to the hair when a peak 5,6-dihydroxyindole (DHI) concentration obtained in the hair dyeing composition is at least about 1.5 mg/ml. This peak DHI concentration, which may be regarded as a practical minimum, occurs typically during the early stage of the reactions described above, normally within the first 30 minutes, preferably within the first 20 minutes,of reactant admixture. An initial dopa concentration of about 3 mg/ml, coupled with DHI molar yield of about 65%, is suitable to achieve the practical minimum peak DHI level in the aqueous composition. It should be understood that the peak DHI concentration is measured during the reactions occurring in the reaction medium and in isolation from the hair, as set forth, for example, in Examples 1-11. As measured by HPLC, molar yields of DHI in accordance with the present invention are typically from about 50 to about 70%, with molar yields of the by-product dihydroxyindole carboxylic acid being from about 7 to 9%, both yields being based on conversion of dopa. Preferably, the peak DHI concentration obtained in the aqueous composition is above about 2.5 mg/ml, most preferably above about 4 mg/ml. DHI molar yields above about 50% and initial dopa concentrations from about 5 mg/ml to the solubility limit in the reaction medium of the dopa species employed are preferred to establish levels of DHI in the hair dye composition suitable to generate a hair dyeing amount of melanin. Of course, the incorporation of the direct dye, and optional primary intermediate and/or coupler hair dye components in the reaction medium will decrease the amount of the 5,6-dihydroxyindole melanin precursor obtained, in favor of other melanin precursors that are not easily quantifiably measured.

For the substituted dopa compounds, a suitable melanin precursor molar yield is typically obtained when the initial substituted dopa concentration is from about 2 mg/ml up to its solubility limit in the reaction medium. Thus, from in vitro experiments it has been found that an initial concentration of alpha methyl dopa of 2 mg/ml yields about 1.5 mg/ml methylindole, which corresponded to about a 90% molar yield. When the initial alpha-methyl dopa concentration was about 8-9 mg/ml, the molar yield was about 60-65%. Similarly, an initial dopamethylester concentration of 2 mg/ml was found to provide dihydroxyindole-2-carboxylic acid methyl ester at about 95% molar yield.

Systems wherein the melanin precursor molar yield and the initial dopa concentration cooperatively provide high melanin precursor concentrations are especially suitable to effect a color change in one treatment in accordance with the present invention, while systems that provide lower melanin precursor concentrations are particularly useful to color hair gradually over successive treatments in accordance with the disclosed process. Typically, 2 to 14 successive treatments for shorter time periods (each less than about 10 minutes, especially less than 5 minutes) are used to color hair gradually.

In the practice of the present invention, the user is provided with two or more containers of reactant-containing solutions, and with printed instructions to mix the solutions in order to form the hair dye composition and to apply the dye composition to the hair for a period of less than about one hour. The process is generally conducted at room temperature, although elevated temperatures obtained by means of a hair dryer, especially in a hair salon, may be used. The user may also place a cap over the hair following the application of the dye composition to the hair, body heat being retained within the cap. Following completion of the contact step, the hair is shampooed to remove excess composition including surface melanin from the hair.

### The Hair Dyeing Kit Product

The kit provided in accordance with this aspect of the invention comprises a sufficient amount of buffer, a first container containing a dopa species solution containing the direct dye or, optionally, the primary intermediate and/or coupler, and a second container containing the oxidant solution. The buffer may be individually packaged in a third container, may be present in the first container, or may be present in the second container. The direct dye, and/or coupler primary intermediate may also be in one or more separate containers.

When the dopa species solution is provided in the form of its acid or basic salt, or is acidic or basic in pH, the buffer would not be present therein. While the kit may contain packets containing amounts, preferably premeasured, of dry powders for preparation of these solutions, it is more convenient to provide them as solutions. Moreover, solutions containing premeasured quantities of the constituents facilitates their correct use by the consumer.

One or more additional containers may be provided in the kit, as described below with regard to optional constituents. The optional constituents may also be contained within the solutions, barring any incompatibility.

The consumer admixes the components of the kit, suitably as the aqueous solutions or as dry powders and water, according to written instructions, to obtain the aqueous reaction mixture. The admixture may be conducted in a separate vessel supplied with or external to the kit, or may take place in a container of the kit adapted to provide sufficient head space for mixing. The reactants may also be admixed on the hair of the user. Essentially upon mixing, reaction of the dopa species will commence. The precursor formed will subsequently oxidize in air to form melanin, visually indicated by the formation of color. The reacting mixture is applied to the hair, the completion of the melanin precursor reaction taking place on the hair, with concurrent diffusion of precursor (and/or partially oxidized precursor) into the hair where the melanin is formed, whereby a permanent hair color is obtained. After the desired hair shade is reached, most preferably within about 30 minutes, the hair dye composition that was applied to the hair is removed, preferably with a conventional shampoo.

Because the hair dye composition is applied to the hair initially or shortly after the reactions commence, the reaction time for melanin formation and the contact time on the hair are essentially the same. The kinetics of melanin formation contemplated by the present invention are such that the reaction should take place within the prescribed contact time constraints previously described. However, failure to remove the hair dyeing composition within the prescribed contact time is not consequential, as no appreciable further hair color change will occur.

### Melanin Promoting Agents

The formation of melanin from the melanin precursor may be promoted by application of a melanin promoting agent or agents, as described below.

Thus, certain transition metal and zinc ions, for example, copper, zinc, nickel, cobalt and iron ions, accelerate the conversion of the melanin precursor to melanin. As used herein "transition metal" is deemed to include zinc. Solutions containing a salt or mixture of salts having these ions when applied to hair in conjunction with the application of the dye composition of this invention to hair result in a deepening of the color obtained. The transition metal salt ions effect a color change to the hair more rapidly than when they are not used. Typically, the color change is obtained in less than about 30, preferably less than about 15 minutes. Because the precursor that is formed is used more efficiently, lower melanin precursor concentrations are suitable in obtaining significant color in a single treatment. Cu⁺⁺ salts and, to a lesser extent, Fe⁺⁺ salts are preferred.

The metal salt solution may be applied to the hair for a predetermined period of time, typically for about 1 to about 10 minutes, before or after treatment with the hair dyeing composition. As a general rule, application of the metal ion solution during the contact of the hair with the hair dye composition is not preferred, as the metal ion causes melanin to form outside the hair shaft. However, in some instances such simultaneous application might be useful, especially with a metal ion agent such as zinc which more slowly effects melanin promotion.

Excess metal salt is removed from the surface of the hair by rinsing or shampooing prior to the application of the hair dye composition. It is suitable to incorporate the metal ions into a shampoo formulation for pre- or post-treatment, in which event a water rinse will suffice to remove the excess. The metal ions are believed to penetrate into the hair shaft and thus be available to rapidly accelerate the conversion of diffused precursor to melanin upon subsequent treatment with the hair dye composition described herein. The metal salt solution typically contains from 0.001 to 1% of the metal salt or salt mixture.

Also suitable to promote melanin formation is an iodide salt when applied in advance of a hydrogen peroxide post-treatment. The iodide may be provided as a 0.01 to 1% solution of the salt, or may be incorporated directly into the hair dye composition. When used as a solution, it may be applied before, during or after treatment of the hair with the hair dye composition. Thereafter, hydrogen peroxide is applied as a 0.1 to 6%, preferably a 1 to 3%, solution.

It is also within the scope of this invention to apply an effective amount of oxidizing solution to the hair as a post-treatment. Suitable oxidizers are, e.g., nitrite, persulfate, periodate, iodate, permanganate and perborate salts in about a 0.1 to 10%, preferably 1 to 5%, aqueous solution.

For best results the agents should be soluble in the aqueous vehicle used in the treatment, and may further contain other adjuvants, such as thickener, surfactant, and the like, e.g., as noted below for the hair dye composition.

Accordingly, the kit containing the first and second premixes may also contain a separately packaged solution of the promoting agent(s). The use of metal salts to enhance the hair color obtained with 5,6-dihydroxyindole is described in British Patent No. 2,132,642, incorporated herein by reference thereto. The use of iodide/peroxide treatment is described in U.S. 4,804,385, and the use of an oxidizing post-treatment is described in U.S. 3,194,734, both patents being incorporated herein by reference thereto.

### Colors

The use of dopa alone as the starting reagent to obtain the melanin precursor 5,6-dihydroxyindole is suitable to produce a melanin that dyes hair black or gray. It is unable to produce chromatic colors. When the substituted dopa compounds are employed, the hair dyeing process of the present invention advantageously dyes hair a range of shades depending upon the selection of the starting substituted dopa species. Thus, colors ranging from light to medium brown to black with red, blue, green and yellow tones are possible, depending on the choice of the starting material and the contact time of the hair dye composition on the hair. Alpha methyl dopa has been found to provide a dark brown color, while medium brown has been obtained with dopa methyl ester, and light brown with epinephrine.

The addition of one or more conventional direct dyes which may be employed together with hair dye couplers or hair dye primary intermediates, to the initial reaction mixture provides a means for introducing chromatic colors to the melanin obtained in the practice of the present invention. Thus, colors ranging from light to medium brown to black with red, blue, green and yellow tones are possible, depending on the choice of the starting materials and the contact time of the hair dye composition on the hair.

The various dopa species suitable for use herein may be used singly or in admixture, alone or in combination with one or more of the oxidative hair dye components, in order to achieve a desired color.

### Hair Dye Constituents

The present invention incorporates one or more direct dyes and, optionally, hair dye primary intermediates and/or hair dye couplers within the reaction medium, with a view towards modifying the ultimate color effect produced on the hair. Thus, it is believed that these conventional hair dye components react with the various species formed during the reaction, thereby incorporating one or more additional chromophoric substituent groups within the ultimate melanin species. The presence of the chromophoric groups provides tonality modification so that a broad array of colors is available to the user. Because the reaction with the hair dye primary intermediates and/or couplers may prevent cyclization, nitrogenous phenolic melanin precursors are likely obtained in lieu of indolic melanin precursors.

The concentration of the direct dyes, couplers and/or primary intermediates is less than about 10 mg/ml, and preferably is present in the reaction medium from about 0.01 to about 5 mg/ml, most preferably from about 0.05 to about 2 mg/ml. The amount of these hair dye components should not be so great as to prevent the formation of indolic melanins, nor should it exceed the concentration of the dopa species. That is, the process of the present invention contemplates reaction of only a portion of the intervening dopa species reaction products with the direct dye or, if present, the primary intermediate and/or coupler compounds. Among these latter compounds, couplers are preferred as they are less likely to be oxidized by the ferricyanide oxidant. Because the ferricyanide will compete for reaction with the primary intermediates, adjustment in ferricyanide concentration and/or primary intermediate concentration might be required.

A wide variety of primary intermediates can be employed in this invention including, for example:
paraphenylenediamines, corresponding to the formula: 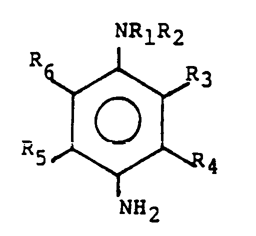 in which R₁ and R₂, which may be identical or different, can denote hydrogen, a C₁-C₆ lower alkyl group, a C₁-C₆ alkyl radical substituted with one or more hydroxy group(s) or with a methoxy, methylsulphonylamino or aminocarbonyl group, a furfuryl group, or a phenyl radical optionally substituted with an amino group; R₃ and R₆ denote, independently of one another, hydrogen, a C₁-C₆ lower alkoxy group, a halogen atom such as a chlorine atom, a with one or more hydroxy group(s), and R₄ and R₅ denote, independently of one another, hydrogen, a C₁-C₆ lower alkoxy group, a C₁-C₆ lower alkyl group, or a halogen atom such as chlorine, as well as their salts with inorganic or organic acids. N,N'-diphenylalkylenediamines in which the phenyl groups are substituted at the para position with an OH or amino group optionally substituted with a C₁-C₆ alkyl group, to be substituted with C₁-C₆ alkyl, C₁-C₆ hydroxyalkyl or C₁-C₆ aminoalkyl; para-aminophenols; ortho-aminophenols; orthophenylenediamines, and heterocyclic oxidation bases.

Among the useful compounds of formula (I), there may be mentioned p-phenylenediamine, 2-methyl-paraphenylenediamine, 2-methoxy-para-phenylenediamine, 2-chloro-N-methyl-paraphenylenediamine, N-furfuryl-para-phenylenediamine, 3-methoxy-N¹-methyl-paraphenylenediamine, 2-chloro-para-phenylenediamine, N-methyl-para-phenylenediamine, 2,3-dimethyl-para-phenylenediamine, 5-chloro-N¹-methyl-p-phenylenediamine, 5-methyl-N¹,N¹-dimethyl-p-phenylenediamine, 5-methyl-N¹-ethyl-N¹-(amino-carbonyl-methyl)-p-phenylenediamine, 5-methyl-N¹-ethyl-N¹-ethylsulphonylamino-ethyl)-p-phenylenediamine, N-(2-methoxyethyl)-p-phenylenediamine, 2,6-dimethyl-p-phenylenediamine, N,N-bis(2-hydroxyethyl)-p-phenylenediamine. The N,N¹-diphenylalkylenediamines include, for example, N,N¹-bis-(2-hydroxyethyl)-N,N¹-bis(p-aminophenyl)-ethylenediamine. Their salts with acids such as the monohydrochlorides dihydrochlorides or sulphates are also suitable.

Among p-aminophenols which are more especially usable according to the invention, there may be mentioned p-aminophenol, 2-methyl-p-aminophenol, 3-methyl-p-aminophenol, 2,3-dimethyl-p-aminophenol, 3-methoxy-p-aminophenol, 2-chloro-p-aminophenol, N-methyl-p-aminophenol and 3-(methylthio)-p-aminophenol, of which p-aminophenol is preferred.

Among ortho bases, ortho-aminophenol, 5-chloro-orthoaminophenol and ortho-phenylenediamine are chosen more especially according to the invention.

Among heterocyclic bases, it is preferable, according to the invention, to use, 2,3-diamino-6-methoxy-pyridine and 2-(2-hydroxyethyl)amino-5-aminopyridine and their salts, and still more especially 3,6-diaminopyridine, 2,6-dimethoxy-3-aminopyridine, 2-methylamino-3-amino-6-methoxypyridine, 2,5-diamino-pyridine, 2-(N-hydroxyethyl)amino-5-amino pyridine, and 2-(N,N-bishydroxyethyl)amino-5-aminopyridine.

More especially preferred primary intermediates are p-phenylenediamine 2-methyl-p-phenylenediamine, N-(2-methoxyethyl)-p-phenylenediamine, N,N-bis(2-hydroxyethyl)-p-phenylenediamine and p-aminophenol.

Among couplers or color modifiers there may be mentioned, in particular, the compounds corresponding to the formula: 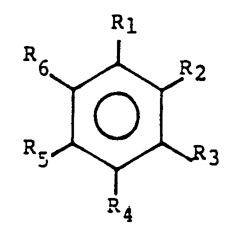 in which R₁ denotes hydroxy or an amino group which can be substituted with one or more C₁-C₆ hydroxyalkyl groups; R₃ and R₅, independently of one another, can denote hydrogen, a hydroxy group, an amino group optionally substituted with a C₁-C₆ lower hydroxyalkyl group or a C₁-C₆ lower alkyl group; and R₂, R₄ and R₆ can denote a hydrogen atom or a C₁-C₆ alkoxy group, a hydroxyalkoxy group or a C₁-C₆ lower alkyl group; it also being possible for R₃ and R₄ together to form a methylenedioxy group.

Among the suitable couplers, there may be mentioned 2-methoxy-5-aminophenol, 2-methoxy-5-[N-(2-hydroxyethyl)amino]phenyl, 1,3-diamino-2,6-dimethoxybenzene, 2-methoxy-1-(N-methylamino)-4-(2-hydroxyethoxy)-3-amino-benzene, 1,3-diamino-6-methoxybenzene, 4,6-dimethoxy-1,3-bis[N-(2-hydroxyethyl)amino]benzene, 2,6-dimethoxy-3-[N-(2-hydroxyethyl)amino]-1-aminobenzene, 2,6-dimethoxy-3-[N-(2-hydroxyethyl)amino]-1-aminobenzene, 2,4 dimethoxy-3-[N-(2-hydroxyethyl)amino]phenol, 1,3-bis[N-(2-hydroxyethyl)amino]-4-methoxybenzene, 3-amino-4-methoxyphenol, 3,4-methylenedioxy-1-aminobenzene, 2,6-dimethyl-3-[N-(2-hydroxyethyl)amino]phenol, 2,6-dimethyl-3-aminophenol, 4-ethoxy-1-amino-3-[N,N-bis(2-hydroxyethyl)amino]benzene, (2,4-diaminophenoxy)ethanol, (2-amino-N-methyl-4-aminophenoxy)ethanol, 1-methoxy-2-[N-(2-hydroxyethyl)amino]-4-aminobenzene, 3,4-methylenedioxy-6-methoxyphenol, 3-amino-6-methylphenol, 3,4-methylenedioxy-6-methoxyaminobenzene, 3-aminophenol, 1,3-dihydroxybenzene-4-(hydroxyethoxy)-1-, 3-phenylenediamine, 4,6-(dihydroxyethoxy)-1-,3-phenylenediamine, and 1,3-phenylenediamine.

Other suitable couplers are 6-aminobenzomorpholine, 1-amino-7-naphthol, 6-hydroxybenzomorpholine, 1-naphthol, 1,3-dihydroxynaphthalene and 1,2-dihydroxybenzene. Among heterocyclic couplers there may be mentioned 2,6-dihydroxypyridine, 2,6-diaminopyridine, 2-amino-4-hydroxypyridine, 2-hydroxy-4-amino-pyridine, 2-hydroxy-5-aminopyridine, 2-amino-6-hydroxypyridine and the like. Included also are further derivatives of 2,6-diamino alkyl pyridines where the amino nitrogen of one or both amino groups is mono- or distributed with a C₁-C₆ alkyl group such as the methyl, propyl, isopropyl, butyl, iso or sec-butyl, pentyl, sec-pentyl neopoentyl, t-butyl, hexyl, 3-methyl pentyl or 4-methylpentyl groups. The amino groups of either the amino-4-hydroxy- or 2-hydroxy-4-amino-pyridines may also have mono- or di-C₁-C₆ alkylation on the nitrogen atoms.

The 2,6 amino-, or 4-amino-2-hydroxy- or 2-amino-4-hydroxy pyridine nitrogens may also either singly or doubly be derivatized with alkoxy substituents of carbon lengths of 1 to 6 with specific mention of 2-hydroxyethyl, 3-hydroxypropyl, 4-hydroxybutyl, 5-hydroxypentyl, 6-hydroxyhexl, 2-hydroxypropyl, 2-hydroxybutyl, 2-hydroxypentyl, 2-hydroxyhexyl, 3-hydroxybutyl, 3-hydroxypentyl, 2-hydroxyhexyl, 4-hydroxypentyl and 5-hydroxypentyl groups.

Among trihydroxylated derivatives of benzene, there may be mentioned 1,2,4-trihydroxybenzene, 1,2,4-trihydroxy-5-alkylbenzenes in which the alkyl group is a C₁-C₆ lower alkyl group and 1,2,3-trihydroxybenzene, and their salts.

Among diaminohydroxybenzenes, there may be mentioned 2,4-diaminophenol and 2,5-diamino-4-methoxy-1-hydroxybenzene, and their salts.

Among aminodihydroxybenzenes, there may be mentioned 2-amino-1,4-dihydroxybenzene, 1,4-dihydroxy-2-diethylaminobenzene and 4-aminoresorcinol, and their salts.

Among substituted 1,2-dihydroxybenzenes, 4-methyl-1,2-dihydroxybenzene and 3-methoxy-1,2-dihydroxybenzene are especially preferred.

The aminohydroxybenzenes are chosen, in particular, from 2-amino-4-methoxyphenol, 2-aminophenol, 4,6-dimethoxy-3-amino-a-hydroxybenzene and 2,6-dimethyl-4-[N-(p-hydroxy-phenyl)amino]-1-hydroxybenzene, and their salts.

By way of a triaminobenzene, there may be mentioned 1,5-diamino-2-methyl-4-[N-(p-hydroxyphenyl)amino]-benzene and its salts.

Also suitable as a coupler is N-acetyl dopa.

The table below lists some of the preferred primary intermediates and couplers for use in this invention.

### Preferred Primary Intermediates and Couplers

- Primary: p-phenylenediamine
- Intermediates:: p-aminophenol
o-aminophenol
N,N-bis(2-hydroxyethyl)-p-phenylenediamine
2,5-diaminopyridine
p-toluenediamine
- Couplers:: resorcinol
m-aminophenol
-naphthol
5-amino-o-cresol
2-methylresorcinol
N-acetyl dopa
4,6-di(hydroxyethoxy)-m-phenylenediamine
m-phenylenediamine

Direct dyes which can be employed in this invention alone or in combination with primary intermediates and/or couplers are colored aromatic nitroamines which as conventionally employed, deposit on the hair and impart a color which is not permanent, i.e. the color is washed out with ordinary shampoos after repeated shampooing. It has now been discovered that certain direct dyes will react with dopaquinone under the reaction conditions of this invention to form covalently bonded compounds which will impart a permanent color to the hair. Such substituted products will effect "permanent" changes in hair color as that term is defined in this disclosure. Such direct dyes will be referred to herein for convenience as "reactive direct dyes".

In one embodiment, the reactive direct dyes are colored primary and secondary aromatic amines substituted with at least one nitro group in which the amino group is sufficiently nucleophilic so that the reactive direct dyes will react with dopaquinone with the formation of a covalent bond. The reactive direct dyes for use in this invention require at least one nitro group for color and at least one nucleophilic amino group for covalent bonding, but there may also be other substituents on the aromatic nucleus. These may be either electronegative or electropositive, the proviso being that such substituents do not so reduce the nucleophilicity of the amino group or otherwise interfere with the amino group, e.g. sterically, so as to render the formation of a covalent bond impossible.

It will be readily apparent to the skilled artisan that there is a large number of reactive direct dyes which will be useful in this invention. Those that are preferred may be represented by the formula: 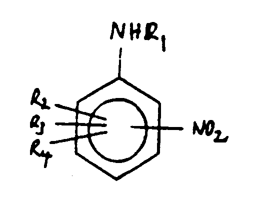 R₁ is H, lower alkyl (C₁-C₆), hydroxyalkyl (C₁-C₆) or phenyl, preferably H;
R₂, R₃, R₄ which may be the same or different are electron donor or acceptor substitutents selected from the group consisting of H, lower alkyl (C₁-C₆), OH, OR, COOR, NHCOR in which R is lower alkyl (C₁-C₆) or hydroxyalkyl (C₁-C₆), CN, COOH, Hal, NO₂, CF₃, SO₃H and NR₅R₆, in which R₅ and R₆ may be the same or different and are selected from the group consisting of H, lower alkyl (C₁-C₆), and substituted lower alkyl (C₁-C₆), in which the substituent may be OH, OR, NHCOR₇, NHSO₂R₇, NHCONH₂, NHCO₂R₇, NHCSNH₂, CN, COOH, SO₃H, SO₂NR₇, SO₂R₇, or COOR₇ in which R₇ is lower alkyl (C₁-C₆), lower hydroxyalkyl (C₁-C₆) or phenyl linked to the nitrogen by an alkylene chain, phenyl or substituted phenyl, with substituents defined as R₂ with the proviso that only one of R₂, R₃ or R₄ can be CN, COOH, Hal, NO₂, CF₃, SO₃H.

Other suitable, although less preferred reactive direct dyes include heterocyclic and fused ring analogs of the above described compounds such as analogs with pyridyl, quinolyl, isoquinolyl, benzofuranyl, isobenzofuranyl, thionaphthyl, isothionaphthyl, indolyl, isoindolyl or naphthyl nuclei.

The presently preferred compounds are
2-Nitro-p-phenylene diamine
4-amino-3-nitrophenol
2-amino-5-nitrophenol
4-N-hydroxyethylamino-3-nitro aniline
2-N-hydroxyethylamino-5-nitro-aniline

### Optional Adjuvant Constituents

The variously described embodiments of the present invention may also include in the hair dye composition one or more optional ingredients, which may be provided in one or more additional containers of the kit for admixture by the user into the aqueous reaction mixture, or, if compatible, may be incorporated into the oxidant or dopa premix solutions described previously.

Well-known conventional additives usually employed in oxidative hair coloring compositions such as organic solvents, thickeners, surface-active agents, pH adjusting agents, antioxidants, fragrances and chelating agents may be included in the compositions of the inventions.

The hair dye compositions used in the process of the present invention can include an organic solvent as a cosolvent. The organic solvent may assist in the dissolution of the components of the composition, and is present typically in an amount up to about 30%, preferably up to about 15%. A desirable range is from about 0.1 to about 15%, most preferably from about 1 to 10%. Suitable solvents are mono- and polyhydric alcohols, for example, ethyl alcohol, isopropyl alcohol, propylene glycol, benzyl alcohol, etc., and glycol ethers, such as 2-butoxyethanol, ethylene glycol monoethyl ether and diethyleneglycol monoethyl ether.

Surface-active agents employed in the dyeing compositions of this invention can be anionic, nonionic, cationic, amphoteric or zwitterionic. By way of examples of the various types of surface-active agents, there can be mentioned: higher alkylbenzene sulfonates; alkylnaphthalenesulfonates; sulfonated esters of alcohols and polybasic acids; taurates; fatty alcohol sulfates; sulfates of branched chain or secondary alcohols; alkyldimethylbenzylammonium chlorides, salts of fatty acids or fatty acid mixtures; N-oxyalkylated fatty acid alkanolamides, and the like. Illustrative of specific surfactants there can be mentioned: sodium lauryl sulfate; polyoxyethylene lauryl ester, myristyl sulfate; glyceryl monostearate; triethanolamine oleate, sodium salt of palmitic methyl taurine; cetyl pyridinium chloride; lauryl sulfonate; myristyl sulfonate, lauric diethanolamide; polyoxyethylene stearate; ethoxylated oleoyl diethanolamide; polyethylene glycol amides of hydrogenated tallow; stearyldimethyl benzyl ammonium chloride; dodecylbenzene sodium sulfonate; triethanolamine salt of p-dodecylbenzene sulfonate; nonylnaphthalene sodium sulfonate; dioctyl sodium sulfonsuccinate; sodium N-methyl-N-oleoyl taurate; oleic acid ester of sodium isothionate; sodium dodecyl sulfate; the sodium salt of 3-diethyl tridecanol-6-sulfate and the like. The quantity of surface-active agent can vary over a wide range, such as from about 0.05% to 15% and preferably from about 0.10 to 5% by weight of the composition. The anionic and nonionic surfactants are employed typically as emulsifiers, while the cationic surfactants are useful to impart a hair conditioning benefit to the hair. Care must be exercised when anionic and cationic surfactants are both incorporated, in view of possible incompatibility.

Chelating and sequestering agents include, for example, ethylenediaminetetraacetic acid, sodium citrate, etc., and are present in an amount of under about 1%.

A thickening agent may also be incorporated in the dyeing composition of this invention, which may be one or several of those commonly used in hair dyeing. These are exemplified by such products as sodium alginate or gum arabic, or cellulose derivatives, such as methylcellulose, e.g., Methocel 60HG, or the sodium salt of carboxymethylcellulose, or hydroxyethyl-cellulose, e.g., Cellosize QP-40 or acrylic polymers, such as polyacrylic acid sodium salt, or inorganic thickeners, such as bentonite. The quantity of this thickening agent can also vary over a wide range, even as high as 20%. Ordinarily it will range from about 0.1 to 5% by weight of the composition. The viscosity of the composition may vary from about 1 cp to about 100,00 cps. For a typical lotion formulation, composition viscosity is between about 100 cps to about 10,000 cps, at which viscosity the composition can be applied to the hair without running or dripping.

The composition of the present invention may also include pH adjustment agents to provide an initial reaction medium pH within the previously stated range. Typically, these pH adjustment-agents are incorporated into dopa species premix, as previously described, to ensure dissolution of the dopa species. However, such pH adjustment agents may also be incorporated into the oxidant premix or directly into the aqueous reaction medium. Typical pH adjustment agents have been described in the section entitled The Dopa Species.

In alkaline solution the dopa salt may be somewhat susceptible to oxidation, for example, by air. Accordingly, a small amount of an antioxidant may be included in the alkaline dopa premix. In such instances the amount of oxidant in the oxidant premix might be increased to neutralize the remaining antioxidant upon admixture of the dopa species and the oxidant premixes.

This list of optional ingredients is not intended as limiting. Other suitable adjuvants for inclusion in the hair dye composition are recited, for example, in Zviak, The Science of Hair Care (1986) and Balsam and Sagarin, Cosmetics: Science and Technology, Vol. 2 (Second Edition 1972).

The invention is now illustrated by the following examples. Unless otherwise indicated, concentrations and ratios in the specification including the examples are on a weight basis by weight of the total composition.

### Example 1

A hair dye composition was provided by mixing a first solution containing 0.2 g DOPA, 0.13 g Na₂CO₃, 0.5 g triethanolamine (TEA) and 0.18 g 2-nitro-p-phenylene diamine in 10 g total with a second solution containing 1.2 g potassium ferricyanide, 0.06 g Na₂CO₃ and 0.02 g citric acid in 10 g total solution. The pH after-mixing was approx. 7-8. The composition was applied to (gray) hair, left for 20 minutes and rinsed off. Afterwards the swatch was exposed to an aqueous solution of NaIO₄ (5%) for 2 minutes, rinsed and dried. A reddish brown color was imparted to the hair.

The color profile of the virgin and treated hair was evaluated using the Hunter Tristimulus method, which method is well known in the art. In the Hunter method, the parameters a and b may be positive or negative and define the chromatic condition of the hair. Thus, the more positive the a value, the greater the greenness of the hair. Similarly, positive b values indicate yellowness, while negative b values indicate blueness. More importantly, the L parameter is a measure of color intensity, and has a value of 0 for absolute black to 100 for absolute white. Generally, hair having an L value of about 15 or less is considered black, while an L value of about 60 is white. It should be understood that the L value scale is not linear, but rather is sigmoidal. Proximate to 0 and proximate to 100 hair color intensity apparent to the human eye varies minimally with unit changes in the L value. Between L values of about 20 to about 50, hair color intensity varies significantly with unit changes in L value. Thus, the Hunter values are more sensitive in the region where the human eye is able to perceive color changes.

| Hunter Tristimulus Values | L | a | b |
|---|---|---|---|
| before dyeing | 39.0 | 0.25 | 6.75 gray |
| after dyeing | 19.45 | 4.24 | 3.93 reddish brown |

### Example 2

The colored swatch from Example I was exposed to 5 cycles of shampooing with a typical commercial shampoo. Hunter Tristimulus Values were measured after each cycle. The color remained essentially unchanged after 5 cycles of shampooing.

| Hunter Tristimulus Values | L | a | b |
|---|---|---|---|
| before dyeing | 19.45 | 4.24 | 3.93 reddish brown |
| after dyeing | 19.64 | 4.59 | 4.20 reddish brown |

The following examples illustrate the dyeing of hair in accordance with the process of the present invention using a coupler and a direct dye simultaneously for a lasting color modification.

### Example 3

A first aqueous hair dye composition was prepared by mixing 0.06 g DOPA, 0.064 g Na₂CO₃, 0.25 g triethanolamine (TEA) and 0.03 g 4-amino-3-nitrophenol in water to a total of 10 g with a second aqueous solution, containing 0.36 g potassium ferricyanide, 0.03 g Na₂CO₃ and 0.01 g citric acid in 10 g total solution. The pH after mixing was apprx. 8.3. The composition was applied to white hair (Piedmont), left for 5 minutes and removed by rinsing. Afterwards the swatch was exposed to an aqueous solution of NaIO₄ (1%) for 2 minutes, rinsed and dried. The hair was dyed to a blond shade

| | Hunter Tristimulus Values | | |
|---|---|---|---|
| dyed hair: | L 45.5 | a 3.5 | b 13.9 |
| undyed hair: | L 67.5 | a-1.0 | b 18.0 |

The color remained essentially unchanged after several cycles of shampooing.

### Example 4

The dyeing composition of Example 3 was prepared and applied to white hair (Piedmont) and allowed to remain for 15 minutes. The dyeing mixture was rinsed off and the swatch was exposed to an aqueous solution of NaIO₄ (1%) for 2 minutes, rinsed and dried.

The hair was dyed to a light brown color.

| Hunter Tristimulus Values | | |
|---|---|---|
| L 33.7 | a 3.3 | b 10.8 |

The color remained essentially unchanged after several cycles of shampooing.

### Examples 5-8

Hair dye compositions were prepared as described in Example 1 utilizing m-amino phenol (m-AP) as a coupler or p-amino phenol (p-AP) as a primary intermediate together with a direct dye of the structure and concentration listed as Modifier 2 in Table I below. Hair was dyed as described in Table I. The results are set forth in Table I . The color of the swatches remained essentially unchanged after several cycles of shampooing.

**Table I**

| Dyeing of bleached (bl) and gray (gr) hair with DOPA (1%), two modifiers and ferricyanide^{a} | | | | | | |
|---|---|---|---|---|---|---|
| No. | Modifier 1 [%] | Modifier 2 [%] | hair | Hunter Tris.Val. | | |
| | | | | L | a | b |
| 5 | m-AP [0.2] | 2-Amino-5-nitro phenol [0.39] | bl | 19.0 | 5.2 | 9.0 |
| | | | gr | 22.1 | 2.5 | 10.5 |
| 6 | m-AP [0.2] | 4-Amino-3-nitro phenol [0.39] | bl | 18.6 | 5.7 | 7.4 |
| | | | gr | 18.8 | 4.3 | 7.2 |
| 7 | m-AP [0.2] | (2-N-hydroxyethyl Amino-5-nitro aniline | bl | 18.4 | 6.3 | 8.3 |
| | | | gr | 22.5 | 2.9 | 10.2 |
| 8 | p-AP [0.25] | 2-nitro-p-phenylene diamine | bl | 20.2 | 8.1 | 8.2 |
| | | | gr | 21.3 | 4.8 | 6.4 |

| | | | | | | |
|---|---|---|---|---|---|---|
| ^{a} Dyeing time 15 minutes; oxidative post-treatment 1% NaIO₄, 2 minutes | | | | | | |

### Example 9 (Comparative)

The ability of an aqueous composition containing dopa and a periodate to form DHI based on the teachings of U.S. Patent 4,746,322 to Herlihy was investigated.

A dopa premix comprising 0.15 g dopa, 0.3 g benzyl alcohol and 10 ml water was prepared by admixture of these ingredients in an open breaker with stirring for about five minutes. 60 mg sodium periodate was then added, with adjustment of the pH to 5.0 with the addition of dilute hydrochloric acid. Aliquots of the solution were removed after 5, 25 and 45 minutes and tested for DHI presence using HPLC techniques. None of the aliquots contained a registrable level of DHI (i.e., less than 0.1 mg/ml DHI).

The experiment was repeated using 60 mg sodium iodate as the oxidant. Again, HPLC analysis failed to show registrable levels of DHI after 5, 25 and 45 minutes.

The following Examples illustrate compositions into which a reactive dye may be incorporated to form hair dyeing compositions for treating hair in accordance with the present invention.

### Examples 10-18

15 ml of a 0.1 M solution of dopa (pH about 1.9) was prepared by dissolving dopa in 0.1 M hydrochloric acid. Also prepared was a 0.36 M solution of potassium ferricyanide also containing a buffer. To form the aqueous reaction medium, equal volumes of the dopa and the oxidant-buffer premixes were combined in a vessel that was open to the atmosphere. The buffer and its concentration in the aqueous reaction medium is indicated in Table II Initial pH values of the reaction medium were measured as noted in Table II The 5,6-dihydroxyindole concentration was measured at 15 minutes following mixing of the premixes, as set forth in Table I, using HPLC techniques known in the art.

**TABLE II**

| Example | Buffer | Buffer Conc. | pH (immediately after mixing) | DHI Conc. at t = 15 min. (mg/ml) |
|---|---|---|---|---|
| 10 | Potassium phosphate | 0.36 M | 6.7 | 4.3 |
| 11 | Potassium phosphate | 0.59 M | 6.8 | 4.5 |
| 12 | Sodium bicarbonate | 0.70 M | 7.0 | 4.6 |
| 13 | Sodium borate | 0.50 M | 7.1 | 1.6 |
| 14 | HEPES | 0.33 M | 7.0 | 3.2 |
| 15 | ACES | 0.33 M | 7.1 | 3.4 |
| 16 | TRIZMA | 0.33 M | 7.5 | 4.8 |
| 17 | TAPS | 0.23 M | 7.7 | 3.4 |
| 18 | TAPS | 0.33 M | 7.6 | 4.1 |

### Example 19

0.15 g dopa was dissolved in 7.5 ml 0.1 M hydrochloric acid to form the dopa premix. An oxidant premix containing 0.9 g potassium ferricyanide, 0.75 g sodium bicarbonate and 7.5 ml water was prepared and rapidly mixed with the dopa premix to form the aqueous reaction medium. The pH of the aqueous reaction medium immediately after mixing was 6.9.

### Example 20

This Example illustrates a reaction medium containing potassium ferricyanide as the oxidant and sodium phosphate as the buffer.

A dopa premix was prepared by adding 0.15 g dopa to 7.5 ml 0.1 M hydrochloric acid. An oxidant premix comprising 0.9 potassium ferricyanide, 1.45 g sodium phosphate (1.15 g Na₂HPO₄ and 0.3 g Na₃PO₄ 12H₂O) and 7.5 ml water was prepared, and rapidly admixed with the dopa premix to provide the aqueous reaction medium, which had an initial pH of 7.2.

### Example 21

This Example illustrates a reaction medium containing potassium ferricyanide as the oxidant and sodium bicarbonate as the buffer.

A dopa premix was formed by adding 0.15 g dopa to 7.5 ml 0.1 M HC1. The oxidant premix contained 0.9 g potassium ferricyanide, 0.87 g sodium bicarbonate and 7.5 ml water, and was admixed rapidly with the dopa premix. The initial pH of the thus formed aqueous reaction medium was 7.1.

### Example 22

Example 20 was repeated except the oxidant premix contained 1.4 g sodium phosphate as the buffer, and further contained 1.79 g sodium citrate to adjust the pH.

### Example 23

Example 20 was repeated except that the buffer was 0.6 g tris(hydroxymethyl)aminomethane.

### Examples 24-25

Hair dye compositions were prepared by mixing a first solution containing 0.15 g dopa, 0.08 g m-aminophenol and 7.5 ml 0.1 M HC1, and a second solution containing potassium ferricyanide in an amount as set forth in Table II, 7.5 ml water and phosphate buffer (1.15 g Na₂HPO₄; 0.3 g Na₃PO₄·12H₂O) to provide an initial pH of the hair dye composition as stated in Table III i.e., as measured after mixing of the first and second solutions.

**TABLE III**

| No. | Oxidant(g) | Initial pH |
|---|---|---|
| 24 | 0.9 | 7.1 |
| 25 | 0.5 | 7.5 |

### Examles 26-28

A mixture of dopa and an amount of m-aminophenol (m-AP) identified in Table IV below was prepared. The hair dye composition contained 0.9 g potassium ferricyanide.

**Table IV**

| No. | m-AP (g) | Initial pH |
|---|---|---|
| 26 | 0.08 | 7.1 |
| 27 | 0.05 | 7.1 |
| 28 | 0.02 | 7.1 |

### Examples 29-33

A hair dye composition was provided by mixing a first solution containing 0.15 g dopa, 0.15 g of a hair dye component identified in Table V below and 7.5 ml 0.1 MHC1, and a second solution containing 0.9 g potassium ferricyanide, sufficient phosphate buffer to provide an initial pH of about 7 and 7.5 ml water.

**Table V**

| No. | Coupler |
|---|---|
| 29 | Resorcinol |
| 30 | 2,6-Dihydroxypyridine |
| 31 | 2,6-Diaminopyridine |
| 32 | 3-Amino-6-(dimethylamino)-methylphenol |
| 33 | 1-Naphthol |

### Example 34

A composition was formed containing 0.075 g dopa; 0.091 g N-acetyl dopa; 0.9 g ferricyanide; sufficient phosphate buffer to provide an initial composition pH of 7.2, and 15 ml water.

### Examples 35-37

Hair dye compositions were prepared by mixing a first solution containing an amount of the dopa species identified in Table VI equal to 0.15 g dopa on an equimolar concentration basis and 7.5 ml 0.1 M HC1, and a second solution containing 0.9 g potassium ferricyanide, 7.5 ml water and sufficient phosphate buffer to provide an initial pH of the hair dye composition of 7.2,i.e., after mixing of the first and second solutions.

**Table VI**

| No. | Dopa Species |
|---|---|
| 35 | α-Methyl dopa |
| 36 | Epinephrine |
| 37 | Dopa methyl ester |

### Examples 38-40

A composition was formed as in Examples 35-37 but wherein dopa was combined with the dopa species identified in Table VII below. The dopa species was present in an equimolar amount to dopa.

**Table VII**

| No. | Dopa Species |
|---|---|
| 38 | Epinephrine |
| 39 | α-Methyl dopa |
| 40 | Dopa methyl ester |

### Examples 41-43

Compositions as in Examples 38 to 40 were prepared but with 0.69% potassium iodide present.

### Examples 44-45

A composition was provided by mixing a first solution contain an amount of a dopa species identified in Table VIII below equal to 0.15g dopa on an equimolar concentration basis, 0.25g meta-amenophenol and 7.5 ml 0.1 M HC1, and a second solution containing 0.9 g potassium ferricyanide, sufficient phosphate buffer to provide an initial pH of 7.2, and 7.5 ml water.

**Table VIII**

| No. | Dopa Species |
|---|---|
| 44 | α-Methyl dopa |
| 45 | Epinephrine |

### Examples 46-47

Same as Examples 44-45 except the composition contained 0.075 g dopa and an amount of the dopa species of Table IX. equal to 0.075 g dopa on an equimolar concentration basis.

**Table IX**

| No. | Dopa Species |
|---|---|
| 46 | α-Methyl dopa |
| 47 | Epinephrine |

## Claims

1. A method of permanently dyeing hair with a melanin precursor characterised in that it comprises the steps of:
(a) forming a melanin precursor by reacting a composition containing a dopa species and a reactive direct dye with a hair dye component which is an oxidant selected from soluble ammonium, alkali metal and alkaline earth metal ferricyanide salts in an aqueous reaction medium containing a buffering agent, the concentrations of the dopa species, reactive direct dye and oxidant reactant being in amounts effective to produce a hair coloring concentration of the melanin precursor in the aqueous reaction medium, said buffering agent being present in the aqueous reaction medium in an amount sufficient to maintain its pH in the range 6 to 10,
(b) contacting the hair with the aqueous reaction medium and allowing the melanin precursor to diffuse into the hair in an amount sufficient to generate a hair coloring amount of melanin, and
(c) permanently coloring the hair by allowing the melanin precursor present in the hair to form melanin.

2. A method as claimed in claim 1 characterised in that the hair dye component additionally contains a hair dye primary intermediate, a hair dye coupler or a mixture thereof.

3. A method as claimed in claim 2 characterised in that the primary intermediate is selected from p-phenylenediamine, p-aminophenol, o-aminophenol N,N-bis(2-hydroxyethyl)-p-phenylenediamine, 2,5-diaminopyridine and p-toluenediamine and the coupler is selected from resorcinol, m-aminophenol, 1-naphthol, 5-amino-o-cresol, 2-methylresorcinol, N-acetyldopa, 4,6-di(hydroxyethoxy)-m-phenylenediamine and m-phenylenediamine.

4. A method as claimed in any preceding claim characterised in that the dopa species is dopa or an acid or basic salt thereof.

5. A method as claimed in any preceding claim characterised in that the dopa species is an alkyl ester of dopa wherein the alkyl group contains 1 to 6 carbon atoms.

6. A method as claimed in any preceding claim characterised in that the buffering agent is selected from ammonium, sodium and potassium salts of phosphates, carbonates, bicarbonates, borates and aminic buffers, and wherein the oxidant is sodium or potassium ferricyanide.

7. A method as claimed in any preceding claim characterised in that the reactive direct dye is selected from compounds having at least one nitro group and at least one nucleophilic amino group.

8. A method as claimed in any preceding claim characterised in that the reactive direct dye is selected from compounds having the structure 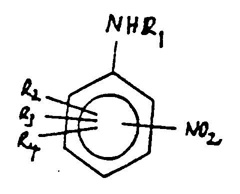 wherein R₁ is H, C₁-C₆ alkyl, hydroxy C₁-C₆ alkyl or phenyl, and R₂, R₃, R₄ which may be the same or different are selected from H, C₁-C₆ alkyl, OH, OR, COOR, NHCOR in which R is C₁-C₆ alkyl or hydroxy C₁-C₆ alkyl, CN, COOH, halogen, NO₂, CF₃, SO₃H and NR₅R₆, in which R₅ and R₆, which may be the same or different, are selected from H, C₁-C₆ alkyl, and substituted C₁-C₆ alkyl, in which each substituent may be OH, OR, NHCOR₇, NHSO₂R₇, NHCONH₂, NHCO₂R₇, NHCSNH₂, CN, COOH, SO₃H, SO₂NR₇, SO₂R₇, or COOR₇ in which R₇ is C₁-C₆ alkyl, hydroxy C₁-C₆ alkyl or phenyl linked to the nitrogen by an alkylene chain, phenyl or substituted phenyl, with substituents defined as R₂ with the proviso that only one of R₂, R₃, or R₄ can be CN, COOH, halogen, NO₂, CF₃ or SO₃H.

9. A method as claimed in any preceding claim characterised in that the reactive, direct dye is selected from 2-nitro-p-phenylenediamine, 4-amino-3-nitrophenol, 2-amino-5-nitrophenol 4-N-hydroxy-ethylamino-3-nitroaniline and 2-N-hydroxy-ethylamino-5-nitroaniline.

10. A method as claimed in any preceding claim characterised in that it further comprises the step of applying to the hair an effective amount of an agent to promote melanin formation.

11. A method as claimed in claim 10 characterised in that the agent is from 0.001 to 1% solution of metal salt selected from copper, zinc, nickel and iron salts and mixtures thereof.

12. A method as claimed in claim 11 characterised in that the metal salt is a copper II salt.

13. A method as claimed in claim 10 characterised in that the agent is an iodide salt solution, said solution being applied to the hair in advance of treatment with a hydrogen peroxide solution.

14. A method as claimed in claim 10 characterised in that the agent is an oxidizing solution applied to the hair as a post-treatment.

15. A hair dyeing kit for permanently dyeing hair with melanin formed from a melanin precursor and which includes in a single package a plurality of containers, characterised in that the kit comprises (a) a first container containing a dopa species and a reactive direct dye in water; (b) a second container containing a water-soluble ammonium, alkali metal or alkaline earth metal ferricyanide salt, and in one of said containers or a separate container a buffering agent selected from ammonium and alkali metal salts of phosphates, carbonates, bicarbonates and borates, and aminic buffers, the amount of buffering agent contained in the kit being sufficient to provide a pH of from 6 to 10 when the contents of the containers are mixed, the dopa and oxidant components in the kit being present in a stoichiometric equivalent ratio of from 1:1 to 2:1 dopa to oxidant.

16. A kit as claimed in claim 15 characterised in that it additionally contains a hair dye primary intermediate, a hair dye coupler or a mixture thereof in the first container or a separate container.

17. A kit as claimed in claim 16 characterised in that the primary intermediate is selected from p-phenylenediamine, p-aminophenol, o-aminophenol N,N-bis(2-hydroxyethyl)-p-phenylenediamine, 2,5-diaminopyridine and p-toluenediamine and the coupler is selected from resorcinol, m-aminophenol, 1-naphthol, 5-amino-o-cresol, 2-methylresorcinol, N-acetyldopa, 4,6-di(hydroxyethoxy)-m-phenylenediamine and m-phenylenediamine.

18. A kit as claimed in claim 15, claim 16 or claim 17 characterised in that the dopa species is as claimed in claim 4 or claim 5.

19. A kit as claimed in any one of claims 15 to 18 characterised in that the buffering agent is selected from ammonium, sodium and potassium salts of phosphates, carbonates, bicarbonates, borates and aminic buffers, and wherein the oxidant is sodium or potassium ferricyanide.

20. A kit as claimed in any one of claims 15 to 19 characterised in that the reactive direct dye is as claimed in claim 7, claim 8 or claim 9.

21. A hair dyeing kit as claimed in any one of claims 15 to 20 characterised in that it further contains an agent to promote melanin formation.

22. A hair dyeing kit as claimed in claim 21 characterised in that the agent is as defined in any one of claims 11 to 14.

## Patentansprüche

1. Verfahren zur permanenten Färbung des Haares mit einem Melanin-Vorläufer, dadurch gekennzeichnet, dass man
(a) durch Umsetzung einer eine Dopa-Verbindung und einen Reaktiv-Direktfarbstoff enthaltenden Zusammensetzung mit einer Haarfärbekomponente, bei der es sich um ein unter löslichen Ammonium-, Alkalimetall- und Erdalkalimetall-Eisen (III)-cyanidsalzen ausgewähltes Oxidationsmittel handelt, in einem einen Puffer enthaltenden wässrigen Reaktionsmedium einen Melanin-Vorläufer bildet, wobei die Konzentrationen der Dopa-Verbindung, des Reaktiv-Direktfarbstoffs und des Oxidationsmittels geeignet gewählt zur Erzeugung einer haarfärbenden Konzentration des Melanin-Vorläufers in dem wässrigen Reaktionsmedium sind und der Puffer in dem wässrigen Reaktionsmedium in einer Menge vorliegt, die zur Einhaltung eines pH im Bereich von 6 bis 10 ausreichend ist,
(b) das Haar mit dem wässrigen Reaktionsmedium in Kontakt bringt und den Melanin-Vorläufer in ausreichender Menge in das Haar diffundieren lässt, um eine haarfärbende Menge Melanin zu erzeugen, und
(c) das Haar permanent färbt, indem man die Bildung von Melanin aus dem im Haar vorliegenden Melanin-Vorläufer ermöglicht.

2. Verfahren nach Anspruch 1, dadurch gekennzeichnet, dass die Haarfärbekomponente zusätzlich eine primäre Zwischenverbindung eines Haarfarbstoffs, einen Haarfarbstoffkuppler oder ein Gemisch davon enthält.

3. Verfahren nach Anspruch 2, dadurch gekennzeichnet, dass die primäre Zwischenverbindung unter p-Phenylendiamin, p-Aminophenol, o-Aminophenol, N,N-Bis(2-hydroxyethyl)-p-phenylendiamin, 2,5-Diaminopyridin und p-Toluoldiamin ausgewählt und der Kuppler unter Resorcinol, m-Aminophenol, 1-Naphthol, 5-Amino-o-cresol, 2-Methylresorcinol, N-Acetyldopa, 4,6-Di (hydroxyethoxy)-m-phenylendiamin und m-Phenylendiamin ausgewählt ist.

4. Verfahren nach einem der vorhergehenden Ansprüche, dadurch gekennzeichnet, dass es sich bei der Dopa-Verbindung um Dopa oder ein saures oder basisches Salz davon handelt.

5. Verfahren nach einem der vorhergehenden Ansprüche, dadurch gekennzeichnet, dass es sich bei der Dopa-Verbindung um einen Alkylester von Dopa handelt, worin die Alkylgruppe 1 bis 6 Kohlenstoffatome enthält.

6. Verfahren nach einem der vorhergehenden Ansprüche, dadurch gekennzeichnet, dass der Puffer unter Ammonium-, Natrium- und Kaliumsalzen der Phosphate, Carbonate, Bicarbonate, Borate und aminischen Puffern ausgewählt ist und es sich bei dem Oxidationsmittel um Natrium- oder Kalium-Eisen(III)-cyanid handelt.

7. Verfahren nach einem der vorhergehenden Ansprüche, dadurch gekennzeichnet, dass der Reaktiv-Direktfarbstoff unter Verbindungen mit wenigstens einer Nitrogruppe und wenigstens einer nukleophilen Aminogruppe ausgewählt ist.

8. Verfahren nach einem der vorhergehenden Ansprüche, dadurch gekennzeichnet, dass der Reaktiv-Direktfarbstoff unter Verbindungen mit der Struktur ausgewählt ist 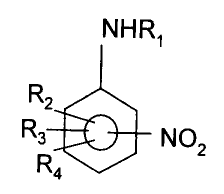 worin R₁ für H, C₁-C₆-Alkyl, Hydroxy-C₁-C₆-alkyl oder Phenyl steht und R₂, R₃, R₄, die gleich oder verschieden sein können, ausgewählt sind unter H, C₁-C₆-Alkyl, OH, OR, COOR, NHCOR, worin R für C₁-C₆-Alkyl oder Hydroxy-C₁-C₆-alkyl steht, CN, COOH, Halogen, NO₂, CF₃, SO₃H und NR₅R₆, worin R₅ und R₆, die gleich oder verschieden sein können, ausgewählt sind unter H, C₁-C₆-Alkyl und substituiertem C₁-C₆-Alkyl, worin es sich bei den Substituenten jeweils um OH, OR, NHCOR₇, NHSO₂R₇, NHCONH₂, NHCO₂R₇, NHCSNH₂, CN, COOH, SO₃H, SO₂NR₇, SO₂R₇ oder COOR₇ handeln kann, worin R₇ für C₁-C₆-Alkyl, Hydroxy-C₁-C₆-alkyl steht oder über eine Alkylenkette an das Stickstoffatom gebundenem Phenyl, Phenyl oder substituiertem Phenyl, worin die Substituenten der Definition von R₂ entsprechen, mit der Maßgabe, dass lediglich einer der Reste R₂, R₃ oder R₄ für CN, COOH, Halogen, NO₂, CF₃ oder SO₃H steht.

9. Verfahren nach einem der vorhergehenden Ansprüche, dadurch gekennzeichnet, dass der Reaktiv-Direktfarbstoff unter 2-Nitro-p-phenylendiamin, 4-Amino-3-nitrophenol, 2-Amino-5-nitrophenol, 4-N-Hydroxyethylamino-3-nitroanilin und 2-N-Hydroxyethylamino-5-nitroanilin ausgewählt ist.

10. Verfahren nach einem der vorhergehenden Ansprüche, dadurch gekennzeichnet, dass man zusätzlich eine wirksame Menge eines die Melaninbildung fördernden Mittels auf das Haar aufbringt.

11. Verfahren nach Anspruch 10, dadurch gekennzeichnet, dass es sich bei dem Mittel um eine 0,001 bis 1 %ige Lösung eines unter Kupfer-, Zink-, Nickel- und Eisensalzen und Gemischen davon ausgewählten Metallsalzes handelt.

12. Verfahren nach Anspruch 11, dadurch gekennzeichnet, dass es sich bei dem Metallsalz um ein Kupfer(II)-Salz handelt.

13. Verfahren nach Anspruch 10, dadurch gekennzeichnet, dass es sich bei dem Mittel um eine Iodidsalzlösung handelt, wobei die Lösung vor der Behandlung mit einer Wasserstoffperoxidlösung auf das Haar aufgetragen wird.

14. Verfahren nach Anspruch 10, dadurch gekennzeichnet, dass es sich bei dem Mittel um eine oxidierende Lösung handelt, die als Nachbehandlung auf das Haar aufgetragen wird.

15. Haarfärbekit zur permanenten Färbung des Haars mit aus einem Melanin-Vorläufer gebildetem Melanin, der in einer Packung mehrere Behälter enthält, dadurch gekennzeichnet, dass der Kit umfasst (a) einen ersten Behälter, der eine Dopa-Verbindung und einen Reaktiv-Direktfarbstoff in Wasser enthält; (b) einen zweiten Behälter, der ein wasserlösliches Ammonium-, Alkalimetall- oder Erdalkalimetall-Eisen(III)-cyanidsalz enthält, und in einem der Behälter oder einem separaten Behälter einen Puffer, der unter Ammonium- und Alkalimetallsalzen der Phosphate, Carbonate, Bicarbonate und Borate und aminischen Puffern ausgewählt ist, wobei die Menge des im Kit enthaltenen Puffers zur Einstellung eines pH von 6 bis 10 ausreicht, wenn die Inhalte der Behälter gemischt werden, und die Dopa- und Oxidationsmittelkomponenten im Kit in einem stöchiometrischen Äquivalentverhältnis von Dopa zu Oxidationsmittel von 1:1 bis 2:1 vorliegen.

16. Kit nach Anspruch 15, dadurch gekennzeichnet, dass er zusätzlich eine primäre Zwischenverbindung eines Haarfarbstoffs, einen Haarfarbstoffkuppler oder ein Gemisch davon im ersten Behälter oder einem separaten Behälter enthält.

17. Kit nach Anspruch 16, dadurch gekennzeichnet, dass die primäre Zwischenverbindung unter p-Phenylendiamin, p-Aminophenol, o-Aminophenol, N,N-Bis(2-hydroxethyl)-p-phenylendiamin, 2,5-Diaminopyridin und p-Toluoldiamin und der Kuppler unter Resorcinol, m-Aminophenol, 1-Naphthol, 5-Amino-o-cresol, 2-Methylresorcinol, N-Acetyldopa, 4,6-Di(hydroxyethoxy)-m-phenylendiamin und m-Phenylendiamin ausgewählt ist.

18. Kit nach Anspruch 15, 16 oder 17, dadurch gekennzeichnet, dass es sich bei der Dopa-Verbindung um eine Verbindung nach Anspruch 4 oder 5 handelt.

19. Kit nach einem der Ansprüche 15 bis 18, dadurch gekennzeichnet, dass der Puffer unter Ammonium-, Natrium- und Kaliumsalzen der Phosphate, Carbonate, Bicarbonate, Borate und aminischen Puffern ausgewählt ist und es sich bei dem Oxidationsmittel um Natrium- oder Kalium-Eisen(III)-cyanid handelt.

20. Kit nach einem der Ansprüche 15 bis 19, dadurch gekennzeichnet, dass es sich bei dem Reaktiv-Direktfarbstoff um eine Verbindung nach Anspruch 7, 8 oder 9 handelt.

21. Haarfärbekit nach einem der Ansprüche 15 bis 20, dadurch gekennzeichnet, dass er zusätzlich ein die Melaninbildung förderndes Mittel enthält.

22. Haarfärbekit nach Anspruch 21, dadurch gekennzeichnet, dass es sich bei dem Mittel um eine Verbindung gemäß Definition in einem der Ansprüche 11 bis 14 handelt.

## Revendications

1. Méthode pour la teinture permanente des cheveux avec un précurseur de mélanine, caractérisée en ce qu'elle comprend les étapes de :
(a) former un précurseur de mélanine par réaction d'une composition contenant une espèce dopa et un colorant réactif direct avec un composant colorant pour les cheveux qui est un oxydant sélectionné parmi des sels solubles de ferricyanure d'ammonium, de métaux alcalins et de métaux alcalinoterreux dans un milieu réactionnel aqueux contenant un agent tamponnant, les concentrations de l'espèce dopa, du colorant réactif direct et de l'oxydant réactant étant en quantités efficaces pour produire une concentration de coloration des cheveux du précurseur de mélanine dans le milieu réactionnel aqueux, ledit agent tamponnant étant présent dans le milieu réactionnel aqueux, en une quantité suffisante pour maintenir son pH dans la gamme de 6 à 10,
(b) mettre les cheveux en contact avec le milieu réactionnel aqueux et permettre au précurseur de mélanine de se diffuser dans les cheveux en une quantité suffisante pour générer une quantité de mélanine colorant les cheveux, et
(c) colorer en permanence les cheveux en permettant au précurseur de mélanine présent dans les cheveux de former de la mélanine.

2. Méthode selon la revendication 1, caractérisée en ce que le composant de coloration des cheveux contient additionnellement un intermédiaire primaire de coloration des cheveux, un coupleur de coloration des cheveux ou un mélange de ceux-ci.

3. Méthode selon la revendication 2, caractérisée en ce que l'intermédiaire primaire est sélectionné parmi p-phénylènediamine, p-aminophénol, o-aminophénol, N,N-bis(2-hydroxyéthyl)-p-phénylènediamine, 2,5-diaminopyridine et p-toluènediamine et le coupleur est sélectionné parmi résorcinol, m-aminophénol, 1-naphtol, 5-amino-o-crésol, 2-méthylrésorcinol, N-acétyldopa, 4,6-di(hydroxyéthyoxy)-m-phénylènediamine et m-phénylènediamine.

4. Méthode selon l'une quelconque des revendications précédentes, caractérisée en ce que l'espèce dopa est dopa ou un sel acide ou de base.

5. Méthode selon toute revendication précédente, caractérisée en ce que l'espèce dopa est un alkyl ester de dopa où le groupe alkyle contient 1 à 6 atomes de carbone.

6. Méthode selon toute revendication précédente, caractérisée en ce que l'agent tamponnant est sélectionné parmi les sels d'ammonium, sodium et potassium de phosphates, carbonates, bicarbonates, borates et tampons aminiques et où l'oxydant est du ferricyanure de sodium ou de potassium.

7. Méthode selon toute revendication précédente, caractérisée en ce que le colorant réactif direct est sélectionné parmi des composants ayant au moins un groupe nitro et au moins un groupe amino nucléophile.

8. Méthode selon toute revendications précédente, caractérisée en ce que le colorant réactif direct est sélectionné parmi des composants ayant la structure 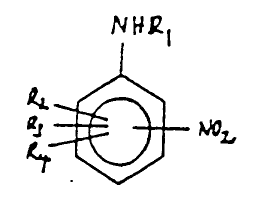 où R₁ est H, alkyle C₁-C₆, hydroxy alkyle C₁-C₆ ou phényle et R₂, R₃, R₄, qui peuvent être identiques ou différents, sont sélectionnés parmi H, alkyle C₁-C₆, OH, OR, COOR, NHCOR où R est alkyle C₁-C₆ ou hydroxy alkyle C₁-C₆, CN, COOH, halogène, NO₂, CF₃, SO₃H et NR₅R₆, où R₅ et R₆, qui peuvent être identiques ou différents, sont sélectionnés parmi H, alkyle C₁-C₆, et alkyle C₁-C₆ substitué où chaque substituant peut être OH, OR, NHCOR₇, NHSO₂R₇, NHCONH₂, NHCO₂R₇, NHCSNH₂, CN, COOH, SO₃H, SO₂NR₇, SO₂R₇ ou COOR₇ où R₇ est alkyle C₁-C₆, hydroxy alkyle C₁-C₆ ou phényle enchaîné à l'azote par une chaîne alkylène, phényle ou phényle substitué avec des substituants définis comme R₂, à condition que seul l'un de R₂, R₃ ou R₄ puisse être CN, COOH, halogène, NO₂, CF₃ ou SO₃H.

9. Méthode selon toute revendication précédente caractérisée en ce que le colorant réactif direct est sélectionné parmi 2-nitro-p-phénylènediamine, 4-amino-3-nitrophénol, 2-amino-5-nitrophénol, 4-N-hydroxy-éthylamino-3-nitroaniline et 2-N-hydroxy-éthylamino-5-nitroaniline.

10. Méthode selon toute revendication précédente, caractérisée en ce qu'elle comprend de plus l'étape d'appliquer aux cheveux une quantité efficace d'un agent pour favoriser la formation de mélanine.

11. Méthode selon la revendication 10, caractérisée en ce que l'agent est une solution à 0,001 à 1% du sel de métal sélectionné parmi le sel de cuivre, de zinc, de nickel et de fer et leurs mélanges.

12. Méthode selon la revendication 11, caractérisée en ce que le sel de métal est un sel de cuivre II.

13. Méthode selon la revendication 10, caractérisée en ce que l'agent est une solution d'un sel d'iodure, ladite solution étant appliquée aux cheveux avant traitement avec une solution de peroxyde d'hydrogène.

14. Méthode selon la revendication 10, caractérisée en ce que l'agent est une solution oxydante appliquée aux cheveux en tant que post-traitement.

15. Nécessaire de teinture des cheveux pour teindre en permanence les cheveux avec de la mélanine formée d'un précurseur de mélanine et qui comprend dans un seul paquet un certain nombre de conteneurs, caractérisé en ce que le nécessaire comprend (a) un premier conteneur contenant une espèce dopa et un colorant direct réactif dans l'eau ; (b) un second conteneur contenant un sel soluble dans l'eau de ferricyanure d'ammonium, d'un métal alcalin ou un d'un métal alcalinoterreux et dans l'un desdits conteneurs ou un conteneur séparé, un agent tamponnant sélectionné parmi les sels d'ammonium et de métaux alcalins de phosphates, carbonates, bicarbonates et borates et des tampons aminiques, la quantité de l'agent tamponnant contenue dans le nécessaire étant suffisante pour produire un pH de 6 à 10 quand les contenus des conteneurs sont mélangés, les composants dopa et oxydant dans le nécessaire étant présents à un rapport stoechiométrique équivalent de 1:1 à 2:1 dopa à oxydant.

16. Nécessaire selon la revendication 15, caractérisé en ce qu'il contient additionnellement un intermédiaire primaire de teinture des cheveux, un coupleur de teinture des cheveux ou un mélange de ceux-ci dans le premier conteneur ou un conteneur séparé.

17. Nécessaire selon la revendication 16, caractérisé en ce que l'intermédiaire primaire est sélectionné parmi p-phénylènediamine, p-aminophénol, o-aminophénol, N,N-bis(2-hydroxyéthyl)-p-phénylènediamine, 2,5-diaminopyridine et p-toluènediamine et le coupleur est sélectionné parmi résorcinol, m-aminophénol, 1-naphtol, 5-amino-o-crésol, 2-méthylrésorcinol, N-acétyldopa, 4,6-di(hydroxyéthoxy)-m-phénylènediamine et m-phénylènediamine.

18. Nécessaire selon la revendication 15, la revendication 16 ou la revendication 17, caractérisé en ce que l'espèce dopa est telle que revendiquée à la revendication 4 ou à la revendication 5.

19. Nécessaire selon l'une quelconque des revendications 15 à 18, caractérisé en ce que l'agent tamponnant est sélectionné parmi les sels d'ammonium, de sodium et de potassium de phosphates, carbonates, bicarbonates, borates et tampons aminiques et où l'oxydant est le ferricyanure de sodium ou de potassium.

20. Nécessaire selon l'une quelconque des revendications 15 à 19, caractérisé en ce que le colorant réactif direct est tel que revendiqué à la revendication 7, la revendication 8 ou la revendication 9.

21. Nécessaire de teinture des cheveux selon l'une quelconque des revendications 15 à 20, caractérisé en ce qu'il contient de plus un agent pour favoriser la formation de mélanine.

22. Nécessaire de teinture des cheveux selon la revendication 21, caractérisé en ce que l'agent est tel que défini selon l'une quelconque des revendications 11 à 14.
